Europäisches Patentamt

European Patent Office

Office européen des brevets

(19)

(11) **EP 1 398 054 A1**

(12) **EUROPEAN PATENT APPLICATION**
published in accordance with Art. 158(3) EPC

(43) Date of publication:
**17.03.2004 Bulletin 2004/12**

(21) Application number: **02705306.5**

(22) Date of filing: **18.03.2002**

(51) Int Cl.⁷: **A61N 1/10**, A61N 1/20

(86) International application number:
**PCT/JP2002/002559**

(87) International publication number:
**WO 2002/081023 (17.10.2002 Gazette 2002/42)**

(84) Designated Contracting States:
**DE FR GB IT**

(30) Priority: **30.03.2001 JP 2001101727**

(71) Applicant: **SHISEIDO COMPANY LIMITED**
**Chuo-ku, Tokyo 104-8010 (JP)**

(72) Inventor: **DENDA, Mitsuhiro,**
**c/o SHISEIDO RESEARCH CENTER**
**Yokohama-shi, Kanagawa 236-8643 (JP)**

(74) Representative: **Merkle, Gebhard**
**TER MEER STEINMEISTER & PARTNER GbR,**
**Patentanwälte,**
**Mauerkircherstrasse 45**
**81679 München (DE)**

(54) **METHOD FOR ACCELERATING THE IMPROVEMENT OF BARRIER FUNCTION OF SKIN**

(57)     The present invention is a method for promoting the improvement of the skin barrier function of the specific skin having low skin barrier function by charging said specific skin with a negative electric potential.

     According to the inventive method, an excellent function promotion effect on the skin having low skin barrier function or reduced skin barrier function is obtained, whereby serving to ameliorate dermal diseases caused by low or reduced skin barrier function.

EP 1 398 054 A1

**Description**

## FIELD OF THE INVENTION

The present invention relates to a method for promoting the improvement of the skin barrier function.

## BACKGROUND OF THE INVENTION

Recently, skin diseases associated with reduced skin barrier function such as atopic dermatitis are increasing. Also increasing recently are cases in which mental stress or skin roughening caused by one's living environmental change the condition of the skin and reduce the skin barrier function. Furthermore, in cases where skin implantation is applied in an orthopedic surgery or a burn surgery, the insufficient formation of a skin barrier layer upon implantation can lead to low or reduced skin barrier function. Thus, low skin barrier function is experienced as a problem in various forms as described above.

While such reduced skin barrier function may undergo spontaneous recovery, a positive clinical treatment is given for example in skin diseases mainly by means of a steroidal anti-inflammatory effect or by moisturizing. However, side effects are known to be associated especially with the former means, which are therefore not successful in improving the barrier itself and are also known to pose a rebound effect if the external application is discontinued. Accordingly, it has been demanded to develop a technology for promoting the improvement of the skin barrier function safely.

In light of the above circumstances, the objective of the invention is to provide a method for promoting the improvement of the skin barrier function.

## SUMMARY OF THE INVENTION

After having committed themselves into researches to solve the above issues, the present inventors discovered that the improvement of the skin barrier function can be promoted by applying a negative potential onto the skin with low skin barrier function and led to the completion of the present invention.

More specifically, the invention is a method for promoting the improvement of the skin barrier function of the specific skin having low skin barrier function by charging said specific skin with a negative electric potential.

In the invention, the improvement of the skin barrier function of the specific skin having low skin barrier function can be promoted by bringing an electrode plate attached to an end of a conductor connected to the negative potential side of a potential generator into contact with a treatment site of said specific skin, loading said specific skin with a negative potential while bringing the electrode plate provided on an end of a conductor, which is earthed and connected to the positive potential side of said potential generator, into contact with a site other than said treatment site, thus causing said other site to be a zero potential.

In the invention, it is preferable that the negative potential is -1.5 to -0.5 V at a direct current.

The specific skin described above may for example be skin which has been damaged by diseases, stress or roughing as well as implanted skin or cultured artificial skin. A representative example of the diseases described above is atopic dermatitis.

In addition, the skin having low skin barrier function is skin with reduced skin barrier function due to skin diseases, mental stress or skin roughing as well as skin with low skin barrier function because of the insufficient formation of a skin barrier layer upon skin implantation or skin with reduced skin barrier function due to implantation. Accordingly, the inventive promotion method encompasses a method for promoting the recovery to the skin having reduced skin barrier function and also for improving the skin having low skin barrier function.

As used herein, the term, "improvement of the skin barrier function" includes the recovery of the skin barrier function.

According to the method of the invention, by loading the specific skin having the low skin barrier function with a negative electric charge, the improvement of the skin barrier function can be promoted.

## BRIEF DESCRIPTION OF THE DRAWINGS

Figure 1 is a schematic view of an example of the device used for promoting the improvement of the skin barrier function according to one embodiment of the invention and an example of the treatment using this device.

Figure 2 is a graph showing the skin barrier function recovery rate measured by the present method.

Figure 3 is an electron microscopic photograph showing the formation of an intercellular lipid in Example 1 together with a comparative electron microscopic photograph.

DESCRIPTION OF THE PREFERRED EMBODIMENTS

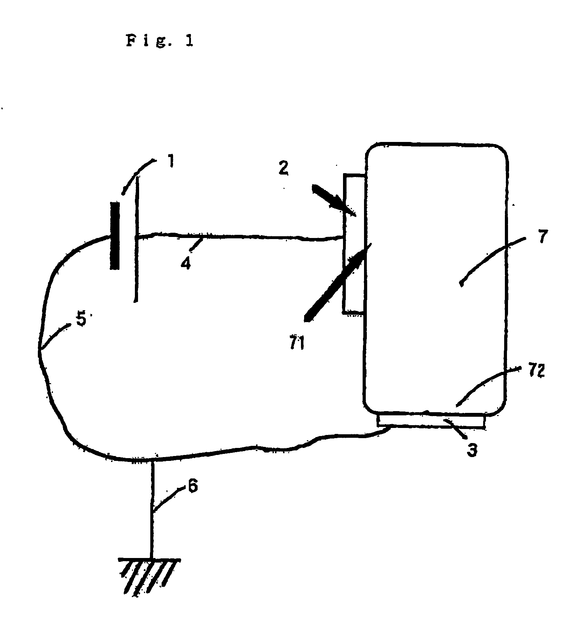

Embodiments of the invention are described below with references to Figures. Figure 1 shows an example of the device for performing the method of the invention, and an example of the treatment using the device. As shown in Figure 1, the device consists of an electric potential generator 1, an electrode plate (A)2 for loading a skin treatment site 71 of skin 7 with the negative electric potential from the electric potential generator 1, and an electrode plate (B) 3 for bringing the positive electric potential from the electric potential generator 1 into contact with said skin at a site 72 which is different from said treatment site 71.

The negative electric potential side of the electric potential generator 1 is connected via a lead 4 to the electrode plate (A)2, while the positive electric potential side of the electric potential generator 1 is connected via a lead 5 to the electrode plate (B)3 and is also earthed via a lead 6. The electric potential generator 1 has a function for transforming the voltage while simultaneously converting an alternating current into the direct current to give any desired direct voltage. Also, while the electrode plates (A) and (B) are not limited particularly, each may for example be in a form of a 2 x 2 cm plate made preferably from a substance which is not dissolved into skin such as gold or carbon, or a substance which has no effect on a living body such as physiological saline.

When the specific skin is subjected to a treatment, the electrode plate (A)2 is brought directly into contact with the site of the skin to be treated, while the electrode plate (B)3 is brought into contact with the site of the skin which is other than the site to be treated, and a negative electric potential is loaded onto the site to be treated via the electrode plate (A)2 from the electric potential generator 1. For the negative electric potential described above, voltage of about -0.5V at a direct current is usually sufficient, with the range from -1.5 to -0.5V being preferred. While the time during which the loading is effected may vary depending on the type of the target skin or the purpose of the treatment, it may be prolonged since the inventive method does not pose any substantial damage to the skin.

Such specific skin to which the present method can be applied may for example be the skin which has been damaged by diseases, mental stress or roughing as well as implanted skin, cultured artificial skin and the like. The stress described above may for example be mental stress, environmental stress exerted for example by soot or cigarette smoke, and UV-light stress from sunlight and the like.

The skin having diseases as mentioned above may for example be skin having atopic dermatitis. Such damaged skin has reduced skin barrier function compared to normal skin.

The cultured artificial skin mentioned above is skin obtained by reconstructing a skin structure using a cell culture technology from a cell of the skin isolated from a living body, such as a multiple cell layer construct consisting of epidermal and dermal layers, or a multiple cell layer construct consisting only of an epidermal layer. The epidermal layer has a structure in which epidemic cell layers are stacked and consists of basal cell layers, prickle cell layers, granule cell layers and corneocyte layers. The cultured artificial skin can be produced for example by incubating a human fibroblast in a collagen gel followed by inoculating and incubating a human keratinization cell onto the gel at the moment of its having shrunk. After incubation, the resultant artificial skin has an insufficiently formed skin barrier layer, and thus has low skin barrier function.

An implanted cell has an insufficiently formed skin barrier layer, and its skin barrier function is low or reduced as a result of the implantation.

The skin barrier function can be measured using as an index a transdermally evaporated water level (hereinafter abbreviated as TEWL). A TEWL can be determined by a method known in the prior art using a commercially available instrument such as an evaporimeter, a tewameter, a MICO water analyzer and the like.

Since the method of the invention can promote the improvement of the skin barrier function, it is useful in medical and cosmetic fields. Also by combining the method with any known technology for improving the skin barrier function, the improvement can further be promoted. For example, the combination with the inhalation of an aroma serves to further promote the effect.

The invention is further described below with specific Examples.

(Example 1)

Using a device shown in Figure 1, a hairless mouse skin was examined for a skin barrier function recovery rate based on the TEWL level. More specifically, hairless mice of 7 to 10 weeks old (HR-1, HOSHINOSHA) were employed and treated as follows. Each hairless mouse was maintained individually in a cage placed in a room at 22 to 25°C and 40 to 70% for at least 4 days before the experiment. The TEWLs of the skin at 2 measurement sites of the left and right flanks of each hairless mouse were measured using a water analyzer (*MICO*) (the TEWL value immediately before the skin barrier function reduction treatment was regarded as A). Then, acetone-soaked cotton was applied to the measurement sites of the skin of each such hairless mouse until the TEWL became about 7 to 10 mg/cm2/h and reduced the skin barrier function, and the TEWL value at that time was measured (the TEWL value immediately after the skin barrier function reduction treatment was regarded as B). At the same time, the gold electrode plates (A) and (B) (2 x 2 cm) of the device shown in Figure 1 were brought into contact with the same sites of the left and right flanks of each hairless mouse. Then the flanks in contact with the gold electrode plate (A) were loaded with -0.5V voltage over a period of 1 hour. 1, 3 and 6 hours after the time at which the skin barrier function was reduced (at the time of TEWL value B), the same respective sites were subjected to the measurement of TEWL (the TEWL value after a certain period following the skin barrier function reduction treatment; C). Based on the measurements described above, a barrier function recovery rate was calculated according to the formula shown below.

$$\text{Skin barrier function recovery rate}(\%)=(B-C)/(B-A)\times100$$

A: TEWL value immediately before skin barrier function reduction treatment

B: TEWL value immediately after skin barrier function reduction treatment

C: TEWL value after a certain period of time following the skin barrier function reduction treatment

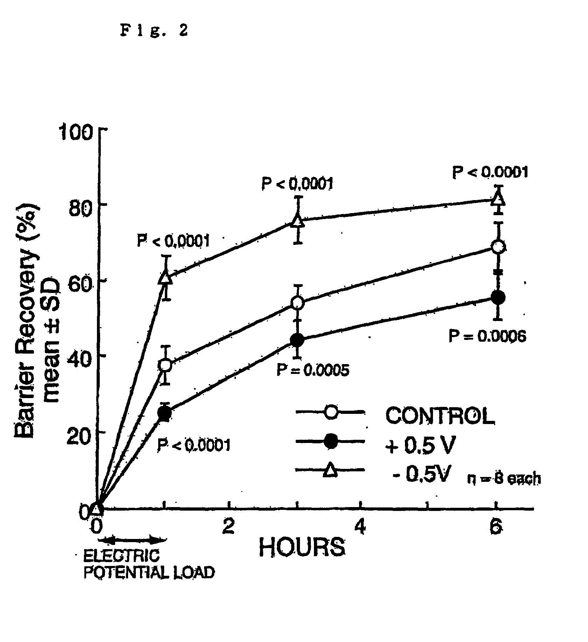

In addition, the TEWL values of the measurement skin sites without loading the potential was leaded with a +0.5V potential and measured similarly for comparison. The result of the skin barrier function recovery rate calculated from each TEWL value is shown in Figure 2 [ordinate: skin barrier function recovery rate (%), abscissa: time]. Each graph represents the mean values of 8 hairless mice respectively.

As shown in Figure 2, the recovery rate after the skin barrier destruction in hairless mice loading -0.5V direct current voltage for 1 hour improved markedly.

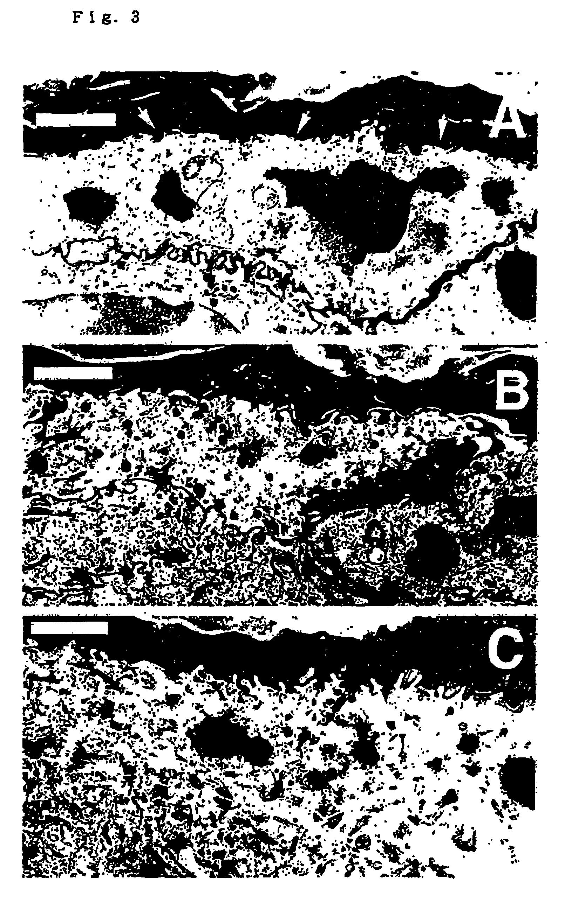

Also, the electron microscopic photograph after 1 hour of loading at -0.5V as described above is shown in Figure 3A, which revealed a marked intercellular lipid (arrow), indicating that the formation (regeneration) of such an intercellular lipid was promoted by loading a negative potential. The electron microscopic photographs with a +0.5V load and without a load were also taken for comparison purpose, and are shown in Figure 3B and 3C. Neither B nor C exhibited any intercellular lipid.

Moreover, the observation of the ion distribution in the skin tissue revealed that the levels of calcium and magnesium which are essential for maintaining the barrier function increased at the surface of the skin as a result of loading a negative potential. Accordingly, a negative potential load is considered to serve for conditioning the behavior of such ions and to lead to the promotion of the barrier recovery.

INDUSTRIAL APPLICABILITY

As discussed above, the method of the invention exhibits an excellent function improvement promotion effect on the skin with reduced or low skin barrier function, whereby serving to ameliorate skin diseases caused by reduced or low skin barrier function.

**Claims**

1. A method for promoting the improvement of the skin barrier function of the specific skin having low skin barrier function by charging said specific skin with a negative electric potential.

2. A method for promoting the improvement of the skin barrier function of the specific skin having low skin barrier function comprising bringing an electrode plate attached to an end of a conductor connected to the negative potential side of a potential generator into contact with a treatment site of said specific skin, loading said specific skin with a negative potential, while simultaneously bringing an electrode plate provided on an end of a conductor, which is earthed and connected to the positive potential side of said potential generator, into contact with a site other than said treatment site if said specific skin whereby promoting the skin barrier function of said specific skin to be a zero potential.

3. A method for promoting the improvement of the skin barrier function according to Claim 1 or 2, wherein the negative potential is -1.5 to -0.5 V at a direct current.

**4.** A method for promoting the improvement of the skin barrier function according to any one of Claims 1 to 3, wherein the specific skin is skin which has been damaged by diseases, stress or roughening.

**5.** A method for promoting the improvement of the skin barrier function according to Claim 4, wherein the disease is atopic dermatitis.

**6.** A method for promoting the improvement of the skin barrier function according to any one of Claims 1 to 3, wherein the specific skin is implanted skin or cultured artificial skin.

Fig. 1

Fig. 2

Fig. 3

## DECLARATION OF NON-ESTABLISHMENT OF INTERNATIONAL SEARCH REPORT
### (PCT Article 17(2)(a), Rules 13*ter*.1(c) and 39)

| Applicant's or agent's file reference<br>SHIS-003-PCT | IMPORTANT DECLARATION | Date of mailing *(day/month/year)*<br>11 June, 2002 (11.06.02) |
|---|---|---|
| International application No.<br>PCT/JP02/02559 | International filing date *(day/month/year)*<br>18 March, 2002 (18.03.02) | (Earliest) Priority Date *(day/month/year)*<br>30 March, 2001 (30.03.01) |

| International Patent Classification (IPC) or both national classification and IPC |
|---|
| Int.Cl7  A61N1/10, A61N1/20 |

| Applicant |
|---|
| Shiseido Co., Ltd. |

This International Searching Authority hereby declares, according to Article 17(2)(a), that **no international search report will be established** on the international application for the reasons indicated below.

1. [X] The subject matter of the international application relates to:

    a. [ ] scientific theories.

    b. [ ] mathematical theories.

    c. [ ] plant varieties.

    d. [ ] animal varieties.

    e. [ ] essentially biological processes for the production of plants and animals, other than microbiological processes and the products of such processes.

    f. [ ] schemes, rules or methods of doing business.

    g. [ ] schemes, rules or methods of performing purely mental acts.

    h. [ ] schemes, rules or methods of playing games.

    i. [X] methods for treatment of the human body by surgery or therapy.

    j. [ ] methods for treatment of the animal body by surgery or therapy.

    k. [ ] diagnostic methods practised on the human or animal body.

    l. [ ] mere presentations of information.

    m. [ ] computer programs for which this International Searching Authority is not equipped to search prior art.

2. [ ] The failure of the following parts of the international application to comply with prescribed requirements prevents a meaningful search from being carried out:

    [ ] the description    [ ] the claims    [ ] the drawings

3. [ ] The failure of the nucleotide and/or amino acid sequence listing to comply with the standard provided for in Annex C of the Administrative Instructions prevents a meaningful search from being carried out :

    [ ] the written form has not been furnished or does not comply with the standard.

    [ ] the computer readable form has not been furnished or does not comply with the standard.

4. Further comments:

| Name and mailing address of the ISA/.<br>Japanese Patent Office | Authorized officer |
|---|---|
| Facsimile No. | Telephone No. |

Form PCT/ISA/203 (July 1998)